# EUROPEAN PATENT APPLICATION

(11) **EP 1 358 895 A1**
(43) Date of publication of application: **05.11.2003**
(21) Application number: 01999395.5
(22) Date of filing: 04.12.2001
(51) Int. Cl.: A61L 27/38

(54) **TISSUE EQUIVALANT FOR TRANSPLANTATION AND PROCESS FOR PRODUCING THE SAME**

(30) Priority: 06.12.2000 JP 2000371692; 28.09.2001 JP 2001302563
(71) Applicant: Japan Tissue Engineering Co., Ltd., Gamagori-shi, Aichi 443-0022 (JP); JAPAN SCIENCE AND TECHNOLOGY CORPORATION, Kawaguchi-shi, Saitama 332-0012 (JP); Ochi, Mitsuo, Hiroshima-shi, Hiroshima 731-0137 (JP)
(72) Inventor: OCHI, Mitsuo, Asaminami-ku, Hiroshima-shi, Hiroshima 7 (JP); UCHIO, Yuji, Izumo-shi, Shimane 693-0023 (JP); KAWASAKI, Kenzo, Izumo-shi, Shimane 693-0051 (JP); KATOH, M, c/o Japan Tissue Engineering Co., Ltd, Gamagori-shi, Aichi 443-0022 (JP); YAMAMOTO, T., c/o Japan Tissue Engineering Co. Ltd, Gamagori-shi, Aichi 443-0022 (JP); FUKUSHIMA,R., c/o Japan Tissue Engineering Co. Ltd, Gamagori-shi, Aichi 443-0022 (JP); KURUSHIMA,T., c/o Japan Tissue Engineering Co. Ltd, Gamagori-shi, Aichi 443-0022 (JP)
(74) Representative: HOFFMANN - EITLE
(86) International application number: JP0110587
(87) International publication number: WO02045766

(57) **Abstract**

A tissue equivalent for transplantation having a three-dimensional structure which is cultured in vitro, contains cells to be transplanted and which can be transplanted into a living body after the culture, characterized by including a scaffold layer mainly culturing a scaffold constituting the three-dimensional structure and a cell layer which is localized at least in a part of the surface of the tissue equivalent for transplantation continuously with the scaffold layer and which contains the cells to be transplanted or extra cellular matrix in a larger amount than the scaffold layer. This tissue equivalent is appropriately employed as a tissue equivalent for transplantation in a relatively large size. This tissue equivalent enables realization of prompt fixation to the neighborhood of the transplanted tissue and prevention of falling off.

## Description

### TECHNICAL FIELD

The present invention relates to a tissue equivalent for transplantation and method for producing same, and particularly to a tissue equivalent for transplantation having a three-dimensional structure that is cultured *in vitro,* contains cells that are to be transplanted and that can be transplanted into a living body after the incubation and method for producing same.

### BACKGROUND ART

Rapid progress has been made in recent years in tissue engineering, whereby treatment is carried out by cell culture and tissue reconstruction. For example, a tissue equivalent for transplantation has been obtained by holding cells on a substrate (scaffold) formed from a variety of highly bio-compatibility materials, and then culturing the cells *in vitro* (outside a living body).

Known tissue equivalents for transplantation include a two-dimensional tissue equivalent that is obtained by seeding and culturing cells on a sheet-like or film-like scaffold, a three-dimensional tissue equivalent that is obtained by culturing cells on a three-dimensional sponge-like (porous body) scaffold. Such tissue equivalents for transplantation have been produced such that the distribution of cells is relatively uniform. That is, when forming a three-dimensional tissue equivalent for the repair of three-dimensional defects such as a cartilage defects, cells are seeded and culturing is carried out so that the cells are also distributed in the thickness direction of the tissue equivalent, which is different from the way in which two-dimensional tissue equivalent are formed.

In the case of three-dimensional tissue equivalents, it is, however, very difficult to form a tissue whereby the cells are distributed relatively uniformly. Particularly when a thick, large tissue equivalent is to be formed, nutrient substances often do not sufficiently spread through to the inside. Thus, log-term culturing or high cell density seeding are being carried out in order to make large tissue equivalents with a relatively uniform cell distribution.

Contrarily, the stiffness of a tissue equivalent is closely related to the cell density and the produced matrix, and therefore even when culturing is carried out so that the cells are distributed relatively uniformly, depending on the transplantation position, there are cases where the cell density is too high and the resulting tissue equivalent becomes too stiff. Such a overly stiff tissue equivalent has less flexibility and even if the tissue equivalent is transplanted in a living body, the tissue equivalent does not have sufficient ability to fuse with the surrounding tissues of the transplanted portion, so that the tissue equivalent may possibly drop off. Conversely, if the cell density is too low, it takes a long time not only to repair the tissue but also to fix the tissue equivalent to the surrounding tissues of the transplanted portion, so that the tissue equivalent may possibly drop off during the time in which it is being fixed.

In view of the above-mentioned problems of the prior art, an object of the present invention is to provide a tissue equivalent for transplantation that enables production of a relatively large tissue equivalent for transplantation, that enables quick fixation of the tissue equivalent to the surrounding tissues of the transplanted portion after transplantation, and that prevents the tissue equivalent from dropping off, and a method for producing same.

### DISCLOSURE OF THE INVENTION

Accordingly, the present invention provides a tissue equivalent for transplantation having a three-dimensional structure which is cultured *in vitro*, contains cells to be transplanted and which can be transplanted into a living body after being cultured, characterized by comprising a scaffold layer mainly constituting a scaffold constructing the three-dimensional structure and a cell layer which is localized at least in a part of the surface of said tissue equivalent for transplantation continuously with said scaffold layer and which contains a large amount of cells to be transplanted or extra cellular matrix than the amount in the scaffold layer.

Since such a tissue equivalent for transplantation of the present invention has a cell layer that is rich in cells to be transplanted or extra cellular matrix on at least a part of the surface thereof, when the tissue equivalent is transplanted, this cell layer comes into contact with the surface of the region of transplantation to thereby increase the compatibility thereof to the surrounding tissue portion after transplantation. In addition, since the tissue equivalent for transplantation of the present invention has a scaffold layer on its inside, the scaffold layer having fewer cells to be transplanted or a smaller concentration of extra cellular matrix than in the above cell layer and mainly constituting a scaffold, appropriate flexibility can be imparted to the tissue equivalent for transplantation to thereby secure the fusion ability with the surrounding tissues of the transplanted portion. Accordingly, even if cells to be transplanted are not distributed homogeneously in the whole tissue equivalent for transplantation, a tissue equivalent having a good bio-compatibility and excelling in the fusion ability with the portion surrounding the transplanted portion can be obtained. After the tissue equivalent for transplantation is fixed to the surrounding tissues of the transplanted portion, since the scaffold works as a base for cell growth and the tissue reconstructs in vivo (in a living body), the portion of transplantation is reconstructed with homogeneous tissue.

Further, the present invention provides a method for producing a tissue equivalent for transplantation having a three-dimensional structure which is cultured *in vitro,* contains cells to be transplanted and which can be transplanted into a living body after the culture, characterized by comprising the steps of embedding cells to be transplanted in said scaffold constructing the three-dimensional structure, supplying a medium in which the cells to be transplanted can be cultured on the scaffold in which the cells to be transplanted are embedded, and culturing the resultant under conditions where the proliferation ratio of the cells to be transplanted is higher on the surface of the above-mentioned scaffold than in the inside of the above-mentioned scaffold, so that the density of the cells to be transplanted becomes higher in at least a part of the surface of the scaffold than in the inside of the scaffold to thereby form two layers having different cell densities.

According to such a method for producing a tissue equivalent for transplantation of the present invention, since two layers having a different cell density are formed by culturing under conditions where the proliferation ratio of the cells to be transplanted becomes high in the surface of the scaffold in which cells to be transplanted are embedded, a layer containing more cells to be transplanted can be formed on the surface of the scaffold and a layer containing fewer cells to be transplanted can be formed in the inside by carrying out culture.

Furthermore, the present invention provides a method for producing a tissue equivalent for transplantation having a three-dimensional structure which is cultured *in vitro,* contains cells to be transplanted and which can be transplanted into a living body after the culture, characterized by comprising the steps of mixing a fluidity scaffold that can maintain a three-dimensional structure in a medium and cells to be transplanted, seeding the mixture obtained by the mixing step on at least a part of the surface of a previously placed three-dimensional scaffold, and culturing the resultant until the cells to be transplanted become substantially dense in at least a part of the fluidity scaffold.

According to this method for producing a tissue equivalent for transplantation of the present invention, a layer having large number of cells to be transplanted can be formed easily on the surface of the scaffold because two layers having different cell densities are formed by seeding cells to be transplanted on at least a part of the surface of the scaffold.

### BRIEF DESCRIPTION OF THE DRAWINGS

Fig. 1 is a sectional view of a tissue equivalent according to an embodiment of the present invention.
Fig. 2 is a sectional view of a tissue equivalent according to another embodiment of the present invention.
Fig. 3 is a sectional view of another tissue equivalent according to a modified example of an embodiment of the present invention.
Fig. 4 is a schematic view illustrating the steps of Example 1 of the present invention.
In Fig. 5, Fig. 5A is a view showing Alcian blue staining of a cross section of a tissue equivalent according to an embodiment of the present invention and Fig. 5B is an enlarged view of Fig. 5A.
In Fig. 6, Fig. 6A is a view showing type II collagen immune staining by antibody of a cross section of a tissue equivalent according to an embodiment of the present invention and Fig. 6B is an enlarged view of Fig. 6A.
Fig. 7 is a schematic view illustrating the steps of Example 2 of the present invention.
Fig. 8 is a photomicrograph with Alcian blue staining of a cross section of the cartilage tissue equivalent according to Example 1 of the present invention.
Fig. 9 is a photomicrograph with Alcian blue staining of a cross section of the cartilage tissue equivalent according to Example 7 of the present invention.
Fig. 10 is a photomicrograph with Alcian blue staining of a cross section of the cartilage tissue equivalent according to Comparative Example 1 of the present invention.
Fig. 11 is a photomicrograph with Alcian blue staining of a cross section of the cartilage tissue equivalent according to Comparative Example 2 of the present invention.
Fig. 12 is a photomicrograph with Alcian blue staining of a cross section of the cartilage tissue equivalent according to Example 8 of the present invention.
Fig. 13 is a photomicrograph with Alcian blue staining of a cross section of the cartilage tissue equivalent according to Comparative Example 3 of the present invention.
Fig. 14 is a graph of the number of viable cells measured in Example 1 and Examples 7 and 8 as well as Comparative Examples 1 to 3.

### BEST MODE FOR CARRYING OUT THE INVENTION

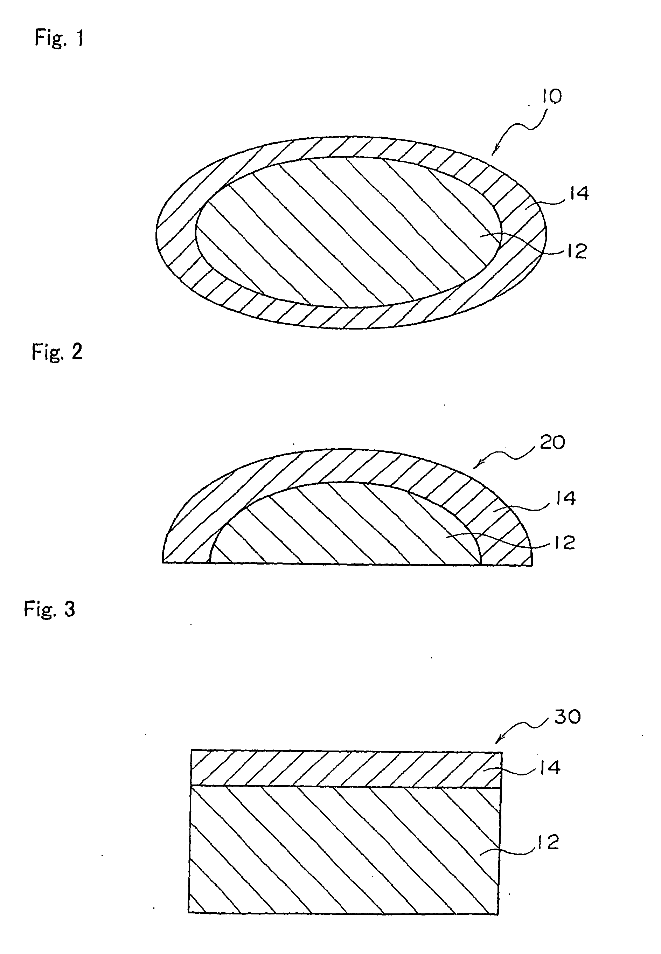

Fig. 1 shows a section of a tissue equivalent 10 according to the present invention. This tissue equivalent 10 is to be transplanted as a graft to a living body. This tissue equivalent 10 contains cells to be transplanted that are the object of the transplantation.

Such a tissue equivalent 10 has a three-dimensional structure composed of a scaffold that is explained hereinbelow. The size of the tissue equivalent 10 differs depending on the size of the portion of transplantation and has a thickness of generally 1 to 10 mm, preferably 2 to 5 mm.

The tissue equivalent 10 includes a scaffold part 12 on the inside and a cell layer 14 on the surface.

The scaffold part 12 is composed of a scaffold that form the three-dimensional structure of the tissue equivalent 10 and cells to be transplanted that are suitable with a tissue into which the tissue equivalent is transplanted. The scaffold part is mainly composed of the scaffold, and fewer cells to be transplanted are distributed therein than in the cell layer 14 that is described below.

The scaffold also has a composition suitable for culture of cells to be transplanted and therefore the cells to be transplanted can grow and proliferate in the scaffold. Although such materials for use in the scaffold can be used if they have a composition suitable for transplantation into a living body, they are preferably bio-degradable materials or bio-compatible materials. Specific examples of these materials include collagen, hyaluronic acid, polyrotaxane, gelatin, fibronectin, heparin, chitin, chitosan, laminin, calcium alginate, polyrotaxane hydrogel, etc. These may be used singly or in combinations of two or more.

The scaffold forms the three-dimensional structure of the tissue equivalent 10 and determines the whole structure of the tissue equivalent 10. The three-dimensional structure of such a scaffold provides a base for proliferation of cells to be transplanted and also provides a culture environment similar to a living body. Therefore a scaffold for use in regeneration of cartilage tissue or bone tissue particularly needs to have a three-dimensional structure. The culture tissue, however, may take any form as long as a three-dimensional structure can be formed. In particular, it is preferably gelled or spongy.

The cell layer 14 is in continuous contact with the outside of the scaffold part 12 and is placed almost all around the periphery tissue equivalent 10. The cell layer 14 is composed of a scaffold, cells to be transplanted, and a produced extra cellar matrix in the same manner as the scaffold part 12, and contains more cells to be transplanted than the scaffold part 12. The cell density of the cells to be transplanted in the cell layer 14 may be substantially dense compared with the cell density of the inside of the scaffold part 12, and is preferably substantially confluent, that is, so dense that adjacent cells exist continuously.

The thickness of the cell layer 14 may be of any thickness as long as a layer of cells to be transplanted is formed on the surface of tissue equivalent 10 and is preferably 20 to 500 µm, particularly 100 to 200 µm. Further, if the thickness is thinner than 20 µm, it is not preferable because the tissue equivalent does not exert enough function as a tissue equivalent 10 containing cells to be transplanted. If the thickness is thicker than 500µm, it is also not preferable because the tissue equivalent may be too stiff as an implant and because the tissue equivalent needs a prolonged culture period, it takes a long time to produce the tissue equivalent, thereby being inefficient.

The cells to be transplanted that can exist in both of the cell layer 14 and scaffold part 12 of the tissue equivalent 10 are cells to be transplanted and can be selected appropriately depending on the portion of transplantation of the tissue equivalent 10. Such a cell for transplantation may be any cell as long as the cell can be transplanted into a living body and is preferably a cell that can proliferate efficiently because of the three-dimensional structure of the tissue equivalent 10. Examples of the cell for transplantation include chondrocyte, osteoblast, fibroblast, adipocyte, hepatocyte, and progenitor cells of these cells. These cells may be used singly or in combinations of two or more depending on the part of the living body to receive the transplantation.

Such a cell for transplantation can be obtained from a living body by known biopsy methods depending on the kind of cell. The obtained cell may be used as it is and in some cases, after the cell is cultured in an appropriate medium for a given period, the resultant cells may be embedded on a scaffold.

In the tissue equivalent 10, the cells to be transplanted produce a extra cellular matrix that is related to proliferation of cells suitable with transplantation and suitability with transplantation environment, and the tissue equivalent 10 therefore may also contain a extra cellular matrix that is produced by these cells as well as the cells to be transplanted. The concentration of the extra cellular matrix depends on the matrix-producing ability of the cells to be transplanted and the number of the cells to be transplanted, and therefore the density is higher in the cell layer 14 than in the scaffold part 12. The extra cellular matrix may be a matrix that is produced by the densely aggregated cells to be transplanted or may be a extra cellular matrix that is added from the outside. These cells to be transplanted and the extra cellular matrix both contribute to the "take" ability of the tissue equivalent 10 to a living body and the stiffness of the tissue equivalent 10.

Accordingly, the tissue equivalent 10 has the cell layer 14 that contains abundant cells to be transplanted or of both cells to be transplanted and a extra cellular matrix on the outermost layer so that the "take" of the tissue equivalent 10 to a living body is high and the tissue equivalent can be fixed for a long period without dropping off. Since the scaffold part 12 positioned inside the tissue equivalent has fewer cells and less extra cellular matrix than the cell layer 14, the tissue equivalent 10 has good flexibility in proportion to the number of cells and the amount of extra cellular matrix. Accordingly, stiffness and flexibility of the whole tissue equivalent 10 can be adjusted to be satisfactory from a bio-compatibility stand point. Moreover, because the tissue equivalent 10 has a cell layer 14 that is rich in surrounding cells and extra cellular matrix, the tissue equivalent 10 is fixed promptly after transplantation and the transplanted tissue equivalent 10 serves as a scaffold after fixation to thereby enable favorable regeneration of tissue in vivo (in a living body).

Next, a method for producing the tissue equivalent 10 will be explained.

The tissue equivalent 10 can be obtained by the steps comprising the steps of embedding the cells to be transplanted in a scaffold, supplying a medium to the scaffold after embedding, and culturing the cells in the medium.

In the step of embedding, cells to be transplanted and a scaffold are mixed by a conventional method (hereinafter referred as the cell-scaffold mixture). At this time, it is appropriate if the seeding cell density of the cells to be transplanted is generally 1x10⁴ cells/ml to 1x10⁸ cells/ml, preferably about 2x10⁴ cells/ml to 2x10⁷ cells/ml, more preferably about 2x10⁵ cells/ml to about 2x10⁶ cells/ml, depending on the planned culture period, the kind of the scaffold, and the composition of the medium. In addition, unless otherwise noted, the seeding cell density represents the density in the state of the cells being embedded in the scaffold. If the seeding cell density is out of the above-mentioned ranges, it is difficult to efficiently form a cell layer 14 that is high in cell density and matrix concentration. Also, in particular, if the seeding cell density is lower than the above-mentioned ranges, it is not preferable because proliferation ratio is insufficient so that it takes a longer period to form a bilayer structure.

In the supply step, a medium in which the cells to be transplanted can be cultured is supplied for the cell-scaffold mixture obtained by embedding the cells in the scaffold.

The basal medium for use in the culture is preferably a liquid medium and any known medium for use in normal culture of the cells to be transplanted can be used as the basal medium that is fundamental to the medium for use in culture. These mediums include, for example, Dulbecco modified Eagle's medium (DMEM) and so on. In the case of a liquid medium, supply is carried out in the following typical manner. A sufficient amount of a liquid medium is poured so that the whole of the cell-scaffold mixture having a three-dimensional structure is under the liquid level. After the cell-scaffold mixture obtained by embedding the cells in the scaffold is placed in an appropriate culture dish as it is, a medium is supplied for this cell-scaffold mixture.

The step of culturing is carried out under conditions where the proliferation ratio of the cells to be transplanted that are embedded in the scaffold becomes higher at the surface of the scaffold. Such high proliferation ratio conditions are selected depending on the kind of cells to be transplanted, the seeding cell density, the composition of the medium, permeability of the medium into the scaffold, and .combinations thereof.

Examples of these high proliferation ratio conditions include selecting a certain kind of serum such as fetal bovine serum (FBS) and a growth factor and the like depending on the kind of the cells to be transplanted, and adding them to the basal medium. For instance, in the case of rabbit chondrocyte, fetal bovine serum (FBS) is preferably used. Furthermore, a high proliferation ratio can be realized by appropriately selecting a seeding cell density that enables prompt transition to the logarithmic growth phase and combinations of such a seeding cell density and a medium that exhibits a high permeability into the scaffold.

Moreover, ascorbic acid is one of the substances that may be added to the basal medium for realizing conditions for a high proliferation ratio. The matrix-producing ability and the proliferation ability of the cell can be improved by adding ascorbic acid to the basal medium. As a result, a larger amount of the matrix is produced by the action of ascorbic acid in the surface layer of the tissue equivalent 10 into which a larger amount of the medium permeates easily compared to the inside of the tissue equivalent 10. Thus, the cell layer 14 that produces a larger amount of the matrix than the scaffold part 12 is formed.

The ascorbic acid used in the present invention may be any L-type ascorbic acid that is normally used in this industry such as salts and hydrates so long as the biological activity of the ascorbic acid is not lost. Examples of such L-type ascorbic acid include stable type ascorbic acids such as L-ascorbic acid-2-phosphate, L-ascorbic acid phosphate ester magnesium salt, L-ascorbic acid-2-sodium sulfate, and 2-O-α-D-glucopiranosyl-L-ascorbic acid (vitamin C-2-glucoside). When ascorbic acid is added to the basal medium in the form of L-ascorbic acid phosphate magnesium salt n-hydrate, the addition amount is 10 to 300 µg/ml, preferably 50 to 200 µg/ml.

Ascorbic acid added to the basal medium, in particular, is preferably maintained in a state where it is preserved by freezing in view of the property that ascorbic acid decomposes in liquid. It is preferred that ascorbic acid is preserved in a frozen state, for example at -5 to -20°C, in a state where ascorbic acid is mixed with the basal medium.

The step of culturing is sufficiently carried out at least during a period in which a bilayer structure is formed. In this period, cells proliferate in the cell-scaffold mixture and the number of the cells in the surface side is different from that in the inside of the tissue equivalent 10 to thereby form the bilayer. The step of culturing is preferably carried out until almost confluent so that the cells constituting the cell layer 14 form a continued dense state in the surface of the cell layer. The cell layer 14 of the tissue equivalent 10 is formed promptly because the step of culturing is conducted under the condition of a high proliferation ratio. The whole area of the surface of the tissue equivalent 10 is in contact with the medium and therefore the culture period can be shortened by, for example, selecting a medium that exhibits a high proliferation ratio. The period necessary for such a bilayer structure is different depending on the seeding cell density and the composition of the medium. For example, when the seeding cell density is 2x10⁶ cells/ml, the period is 2 to 3 weeks.

The cell layer 14 of the tissue equivalent 10 is positioned almost all around the outer periphery of the scaffold part 12. To obtain a tissue equivalent 10 having a cell layer 14 around the outer periphery, the tissue equivalent 10 is first peeled off from the culture surface of culture dish during the culture period. The tissue equivalent 10 peeled off from the culture dish floats in the medium and therefore by continuing the step of culturing under this condition, the cell layer 14 is formed around the outer periphery that is in contact with the medium.

Consequently, according to the present invention, a tissue equivalent 10 that was an excellent "take" rate and that exhibits superior fixation to a living body can be produced efficiently without having to consider the cell density of the inside of the tissue equivalent, and even if the number of the available cell is small, a tissue equivalent having a large three-dimensional structure can be provided with comparative ease. Moreover, in the case of the tissue equivalent 10, the cells do not need to be distributed homogeneously in the inside of the equivalent, so that the tissue equivalent can be produced more promptly compared with an tissue equivalent in which the cells are distributed homogeneously.

Such a tissue equivalent 10 is excellent in the fusion ability with the surrounding tissues of the transplanted portion and therefore the equivalent is fixed to the surrounding tissues of the transplanted portion comparatively promptly, so that the possibility that the tissue equivalent drops off can be decreased.

In addition, in the case of the tissue equivalent 10, the cells to be transplanted and the scaffold are mixed in the step of embedding, and the scaffold part 12 also contains cells to be transplanted. However, if the cell layer 14 contains cells or a extra cellular matrix, the scaffold part 12 may not contain any cells. Such a tissue equivalent having a scaffold part 12 containing no cells can be obtained by previously placing a scaffold in an culture dish, and then seeding a cell-scaffold mixture on the surface of the scaffold and initiating the culture.

The scaffold for use in the above process may be gelled or spongy as that described above. If the scaffold is mixed with the cells to be transplanted, the scaffold requires fluidity. By the use of such a scaffold having fluidity, the cells to be transplanted need only to be placed or applied on the surface of a scaffold that is previously placed in an culture dish, to appropriately cover the surface of the scaffold. The culture period may be a period during which the cells to be transplanted proliferate to be confluent at least in a part of the fluidity scaffold or a period during which the cells produce an appropriate amount of the extra cellular matrix. That is why a bilayer structure can be formed for a short period by a comparatively easy operation. Accordingly, the tissue equivalent 20 having a three-dimensional structure that excels in the fusion ability with a living body can be produced easily.

Furthermore, the tissue equivalent 10 has the cell layer 14 around the outer periphery thereof. The cell layer 14 can exert its effect in improvement in fixation of the tissue equivalent so long as the cell layer 14 has been formed on the contact surface of the transplanted portion thereof with the living body during transplantation. Consequently, the cell layer 14 does not need to exist around the whole outer periphery of the tissue equivalent so long as the cell layer 14 is formed at least on a part of the surface of the tissue equivalent.

Fig. 2 shows another tissue equivalent 20. The tissue equivalent 20 has a part where a cell layer 14 is not formed on a part of the outer periphery thereof. This tissue equivalent 20 does not need the cell layer 14 to be formed around the whole outer periphery, so that the tissue equivalent can be made in a shorter period compared with the case where the cell layer is formed around the whole outer periphery thereof.

Such a tissue equivalent 20 can be obtained easily by finishing the step of culturing without removing the tissue equivalent from the culture dish after initiating culture in the incubator.

In addition, Fig. 3 shows a modified example of another embodiment of the tissue equivalent 30. In this tissue equivalent 30, the portion where the cell layer 14 is formed is narrower than the portion where the scaffold part 12 is exposed. Such a tissue equivalent 30 is transplanted so that the cell layer 14 that is formed on a part of the whole outer periphery is in contact with transplanted portion of the living body. In this case, the same effect as described above can be obtained because the cell layer 14 that is in contact with transplanted portion of the living body can contribute to fixation thereof to the living body.

Such a tissue equivalent 30 can be obtained easily by cutting the tissue equivalent 20 described above in the thickness direction. Thus the shape of the tissue equivalent 30 can be adjusted to the shape of the portion of transplantation and therefore the adherence to the transplanted portion of living body can be improved. Accordingly, fixation to the living body and tissue regeneration can be realized much more promptly and the tissue equivalent can be prevented from dropping off.

Hereinafter, the details of the present invention will be specifically explained by reference to the following examples, but the present invention is not limited to these examples.

### EXAMPLES

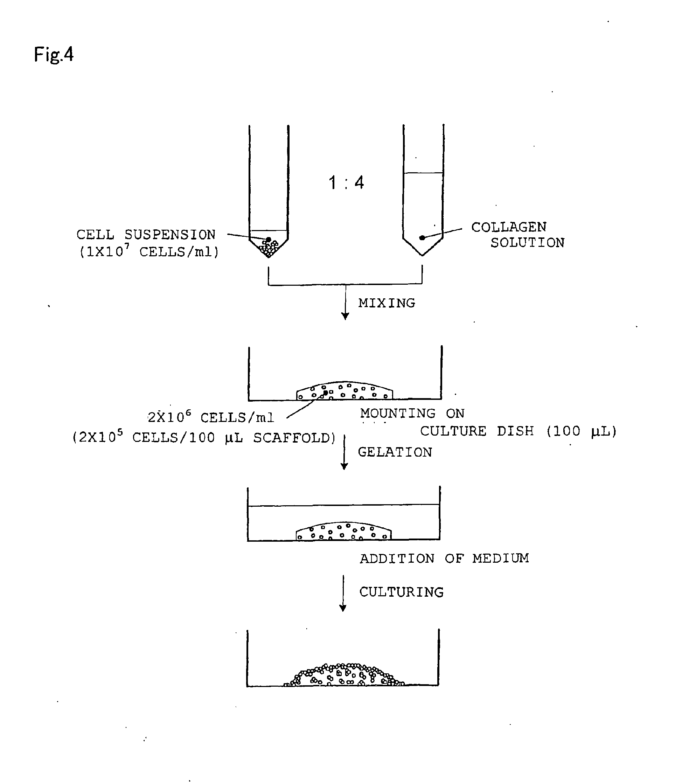

### <Example 1> (See Fig. 4)

Articular cartilage obtained from the knee joint, hip joint, and shoulder joint of a Japanese white tame rabbit, was digested by an enzymatic treatment with a trypsin-EDTA solution and a collagenase solution to thereby separate and collect chondrocyte. The obtained chondrocytes were rinsed,. followed by addition of a 10% fetal bovine serum (FBS)/DMEM medium to thereby prepare a cell suspension with a cell density of 1x10⁷ cells/ml. One part by volume of the cell suspension was mixed (embedded) with four parts by volume of a 3% Atelocollagen Implant (available from Koken Co., Ltd.), and 100 µl of the resulting mixture was mounted (placed) in a dome shape on a culture dish. Since the cell density is diluted by this step, when the cell suspension is prepared with a cell density of 1x10⁷ cells/ml, the density of the seeded cells at the time of embedding the collagen was 2x10⁶ cells/ml (2x10⁵ cell/100 µl scaffold).

The mounted mixture was gelled by allowing it to stand under the condition of 37°C in a 5% CO₂ atmosphere for 0.5 to 1 hour, followed by addition of a medium and initiation of cultivation. The medium used here was a 10 v/v% FBS (fetal bovine serum)-DMEM (Dulbecco modified Eagle's medium) prepared so as to contain 50 µg/ml of ascorbic acid (L-ascorbic acid phosphate magnesium salt n-hydrate: C₆H₆O₉P·3/2Mg·nH₂O; available from Nikko Chemicals Co., Ltd.) and 100 µg/ml of hyaluronic acid. The chondrocytes were thus cultured under the condition of 37°C in a 5% CO₂ atmosphere for 3 weeks.

After 3 weeks of culture, a tissue equivalent having a diameter of about 10 mm and a thickness of about 3 mm was obtained. To identify the morphology of this tissue equivalent, Alcian blue staining for acidic mucopolysaccharides [GAG] which are extra cellular matrixes produced by chondrocyte and Kernechtrot staining for the cell nucleus were carried out by the conventional methods. Subsequently, a cross section of the tissue equivalent was observed with a microscope and it was found that the cell density was not homogeneous and a two layers of a cell layer and a scaffold layer was formed.

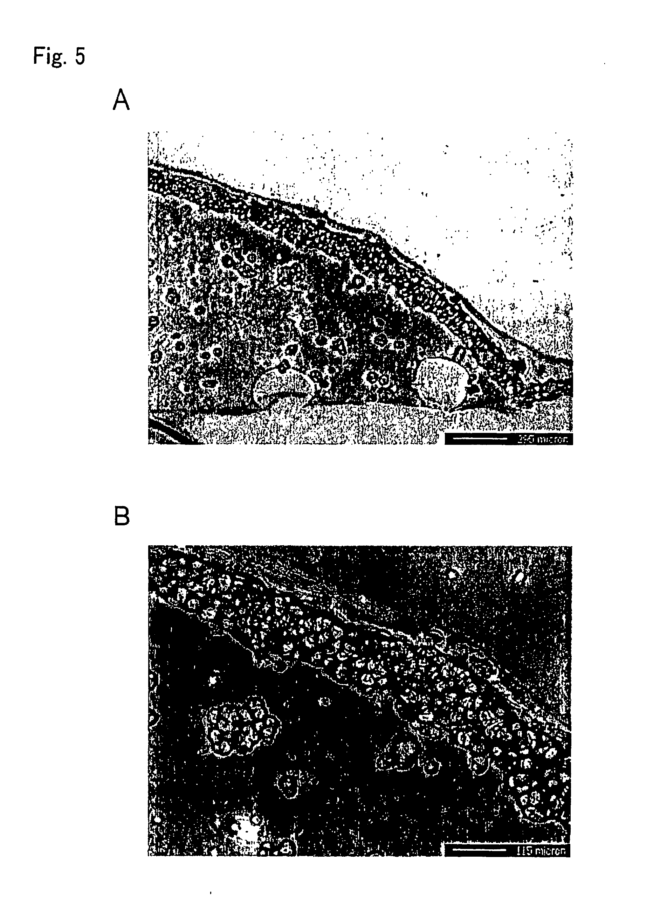

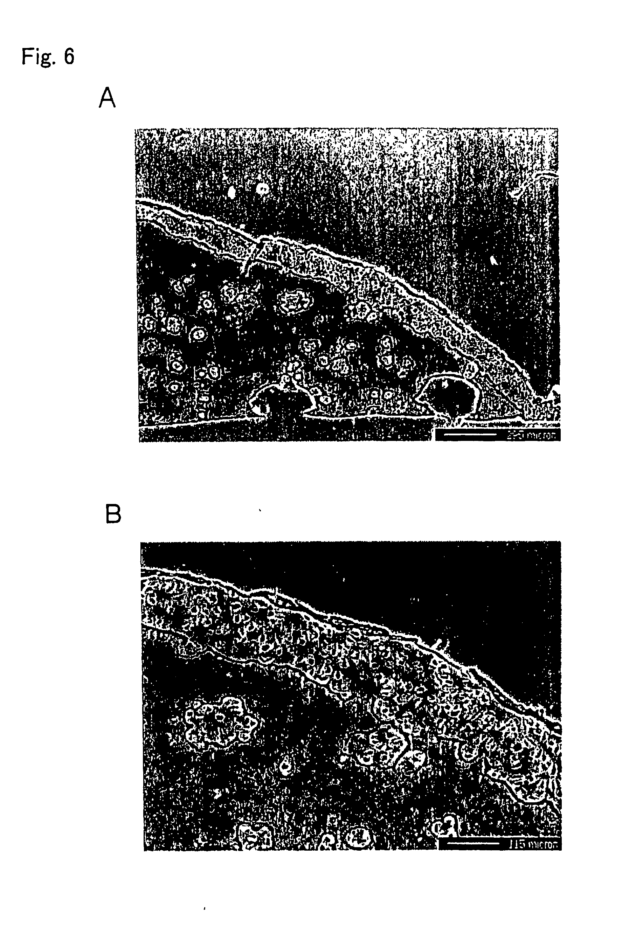

Figs. 5A and 5B are photomicrographs of a cross section of cultured cartilage tissue stained with Alcian blue after 3 weeks of culture. As shown in Fig. 5, produced acidic mucopolysaccharides [GAG] densely concentrated on the surface of the collagen gel and only some colonies are formed in the inside of the gel. In this case, the thickness of the cell layer was about 100 to 200 µm. In addition, the same results were obtained also from the photomicrographs of the stained type II collagen, which is a characteristic extra cellular matrix produced by chondrocyte (Figs. 6A and 6B).

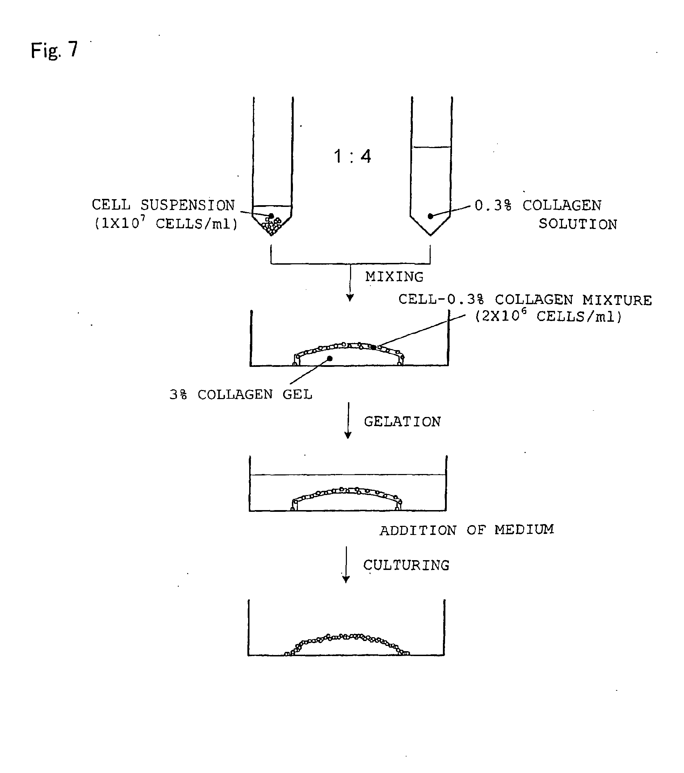

### <Example 2> (See Fig. 7)

Chondrocytes were separated and collected in the same manner as in Example 1. Then, 3% Atelocollagen Implant (available from Koken Co., Ltd.) was mounted (placed) in a dome shape in a culture dish. After the mounted collagen was gelled, a cell-0.3% collagen mixture (2x10⁶ cells/ml) obtained by mixing one part by volume of a cell suspension prepared so as to have a cell density of 1x10⁷ cells/ml and four parts by volume of a 0.3% Atelocollagen solution was seeded as a surface layer on the gelled collagen. After the seeded mixture was gelled, a medium was added and culture was started. The medium used here was a 10 v/v% FBS-DMEM (Dulbecco modified Eagle's medium) prepared so as to contain 50 µg/ml ascorbic acid (the same L-ascorbic acid phosphate magnesium salt n-hydrate as used in Example 1 and the same refers to those mentioned hereinbelow) and 100 µg/ml hyaluronic acid. The chondrocytes were thus cultured at 37°C in a 5% CO₂ atmosphere for 3 weeks. After 3 weeks of culture, a tissue equivalent having a diameter of about 10 mm and a thickness of about 3 mm was obtained. To identify the production of extra cellular matrix by chondrocytes in this tissue equivalent, Alcian blue staining, which is a detection method of acidic mucopolysaccharides, was carried out by the conventional method. Consequently, it was identified that produced acidic mucopolysaccharides densely concentrated on the surface of the collagen gel in a similar manner as in Example 1.

On the one hand, chondrocytes densely concentrated and extra cellular matrix existed abundantly on the surface of the tissue equivalent obtained here. On the other hand, no cells and no extra cellular matrix were found in the inside. Accordingly, it was found that the tissue equivalent had an appropriate stiffness because the tissue equivalent was transformed moderately when a load was put in the thickness direction.

### <Examples 3 to 6>

Culture was started in the same manner as in Example 1 except that the seeding cell density was changed so as to be 2x10³ cells/100 µl scaffold (Example 3), 2x10⁴ cells/100 µl scaffold (Example 4), 2x10⁵ cells/100 µl scaffold (Example 5), and 2x10⁶ cells/100 µl scaffold (Example 6), respectively. The number of the cells and the morphology of the tissue equivalent were examined every 7 days. The results are shown in Table 1.

In Table 1, x represents the case where the bilayer structure was scarcely observed, Δ represents the case where the bilayer structure was partially observed, and ○ represents the case where the bilayer structure was observed, respectively.

As can be seen from these results, a tissue equivalent can be obtained after culture for a given period with any of the above-mentioned seeding cell densities. In addition, the bilayer structure was formed on a part of the surface of the tissue equivalent on about the 10th day when seeding was carried out with a seeding cell density of 2x10⁵ cells/100 µl scaffold, and within 10 days when seeding was carried out with a seeding cell density of 2x10⁶ cells/100 µl scaffold. Accordingly, the tissue equivalent can be used promptly as a graft.

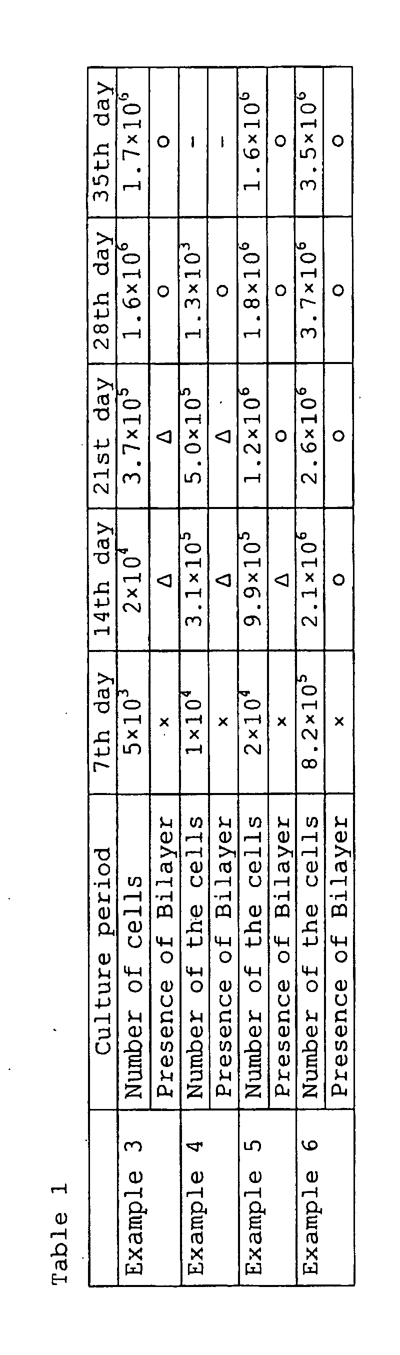

### <Example 7, Comparative Examples 1 and 2>

Culture operation was carried out in the same manner as in Example 1 except that 10% v/v FBS-DMEM was used as a basal medium and a medium to which only 50 µg/ml of ascorbic acid (L-ascorbic acid phosphate magnesium salt n-hydrate) was added (Example 7), a medium to which only 100 µg/ml hyaluronic acid was added (Comparative Example 1), and a basal medium to which no additives were added (Comparative Example 2) were used as a medium for culture, respectively.

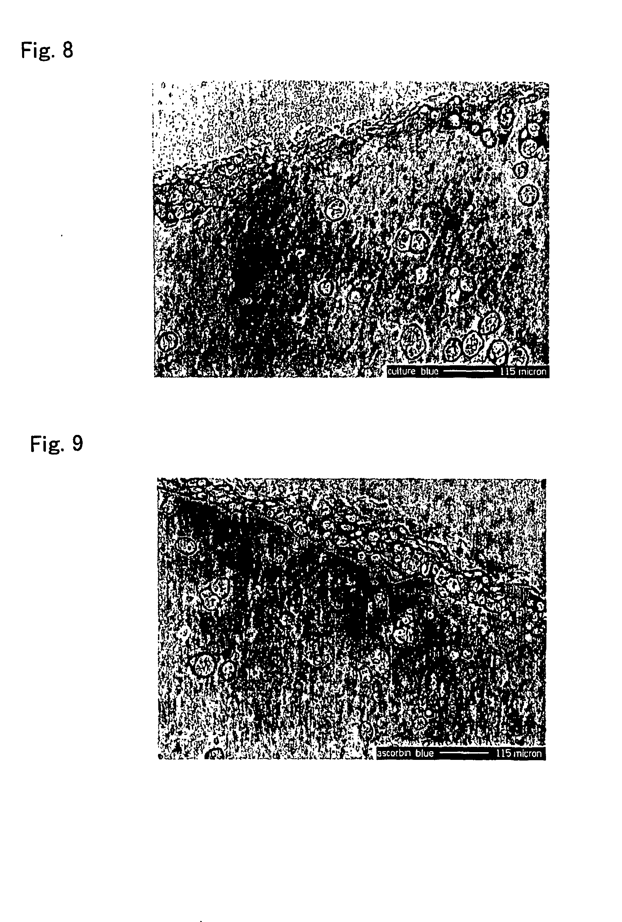

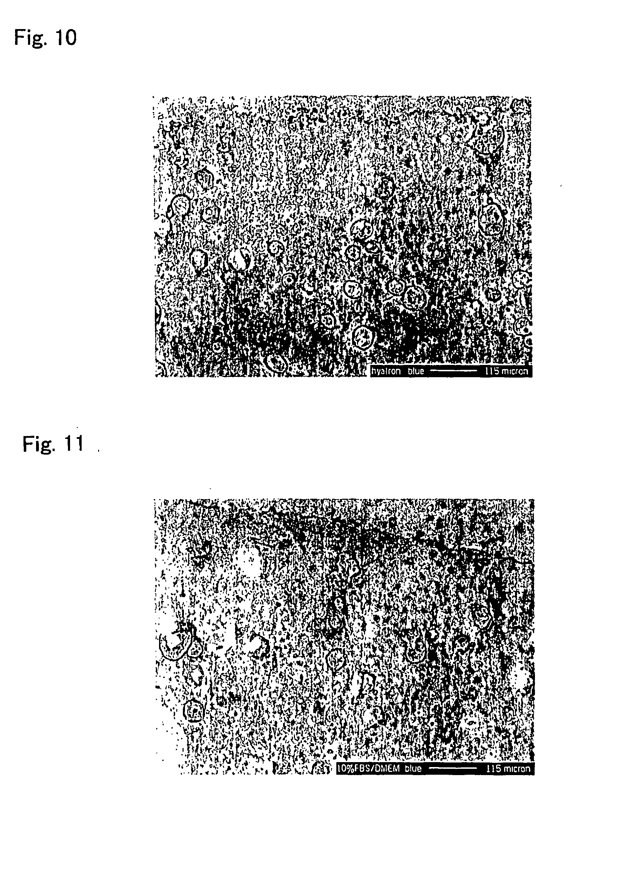

Fig. 8 is a photomicrograph of a cross section of cultured cartilage tissue stained with Alcian blue after 3 weeks of culture under the condition of Example 1, Fig. 9 is that of Example7, Fig. 10 is that of Comparative Example 1, and Fig. 11 is that of Comparative Example 2. As can be seen from the results of the cross-sectional photomicrographs, in Example 1 and 7 (Figs. 8 and 9) in which ascorbic acid was added as an additive, it can be confirmed that a cell layer was formed on the surface of the cultured cartilage tissue to thereby form a bilayer structure. On the other hand, no cell layer was formed in Comparative Example 1 and 2 (Figs. 10 and 11).

Table 2 shows whether the bilayer structure of the cultured cartilage tissue is present or not, extra cellular matrix production, and the number of cells in Examples 1 and 7 and Comparative Examples 1 and 2 on the basis of the cultured cartilage tissue that was produced in Example 1. In Table 2, "Control" represents a standard against which the extra cellular matrix production and the number of the cells in Example 7 and Comparative Examples 1 and 2 are compared with those in Example 1.

**Table 2**

| | Bilayer structure | Extra cellular matrix production | Number of the cells |
|---|---|---|---|
| Example 1 | Present | Control | Control |
| Example 7 | Present | Almost the same | Almost the same |
| Comparative Example 1 | None | Less | About 1/10 |
| Comparative Example 2 | None | Less | About 1/10 |

As seen from the experimental results, it was found that the cell layer could be formed efficiently on the surface of the cultured cartilage tissue by adding ascorbic acid to the basal medium under a culture condition such as that of Example 1.

### <Example 8, Comparative Example 3>

A 10 v/v% FBS-DMEM containing 50 µg/ml ascorbic acid, (L-ascorbic acid phosphate magnesium salt n-hydrate) and 100 µg/ml hyaluronic acid was used as a medium for culture. The ability of the cultured cartilage tissue to form a cell layer was compared between a medium that was obtained by preserving the above-mentioned medium at a low temperature of 2 to 8°C for a long period (Comparative Example 3) and a medium that was obtained by cryopreserving the above-mentioned medium in a frozen state at -5°C for a long period (Example 8).

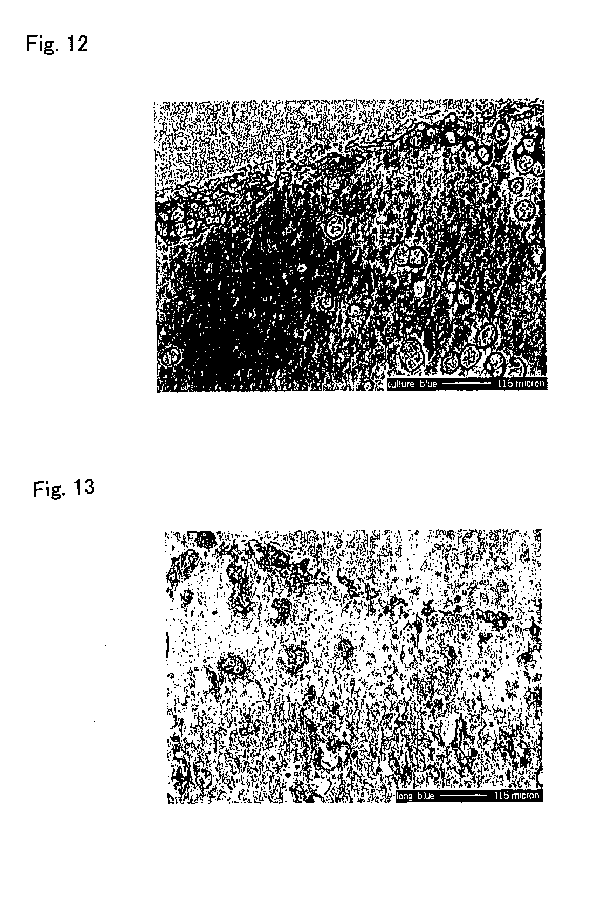

Culture was carried out in the same manner as in Example 1. However, the above-mentioned culture medium that was prepared at the start of culture was preserved at a low temperature or cryopreserved in a frozen state and only a required amount of each medium was returned from the preserved state to the state for use and was used when the medium was to be replaced. The tissue was cultured for 3 weeks. In addition, in Example 1, a medium was prepared freshly at every replacement of the medium and the old medium was removed. Fig. 12 is a photomicrograph of a cross section of a cultured cartilage tissue stained with Alcian blue that was cultured in the medium for cryopreservation in a frozen state (Example 8) and Fig. 13 is a photomicrograph of a cross section of a cultured cartilage tissue stained with Alcian blue that was cultured in the medium for preservation at a low temperature (Comparative Example 3).

Table 3 shows whether the bilayer structure of the cultured cartilage is present or not, extra cellular matrix production, and the number of cells in Example 8 and Comparative Example 3 on the basis of the cultured cartilage that was produced in Example 1. In Table 2, "control" represents a standard against which the extra cellular matrix production and the number of the cells in Example 8 and Comparative Example 3 are compared with those in Example 1.

**Table 3**

| | Bilayer structure | Extra cellular matrix production | Number of the cells |
|---|---|---|---|
| Example 1 | Present | Control | Control |
| Example 8 | Present | Almost the same | Almost the same |
| Comparative Example 3 | None | Less | About 1/6 |

From these experimental results, a cell layer was formed in the medium for cryopreservation in a frozen state and a cell layer was barely formed in the medium for preservation at a low temperature. Since ascorbic acid has a characteristic in that it oxidizes to decompose or hydrolyzes in liquid, it is presumed that when it was preserved in a liquid state, its activity gradually decreased to thereby form no cell layer. On the other hand, it is presumed that any decrease in the activity of ascorbic acid was inhibited in the case of preservation in a frozen state, and therefore the cell layer was formed.

As described above, there is a possibility that a cultured cartilage having no cell layer is formed in spite of a medium containing ascorbic acid because the activity (effect) of ascorbic acid varies depending on the preservation method. Accordingly, it turns out that the activity of ascorbic acid in a medium is greatly involved in the formation of the cell layer.

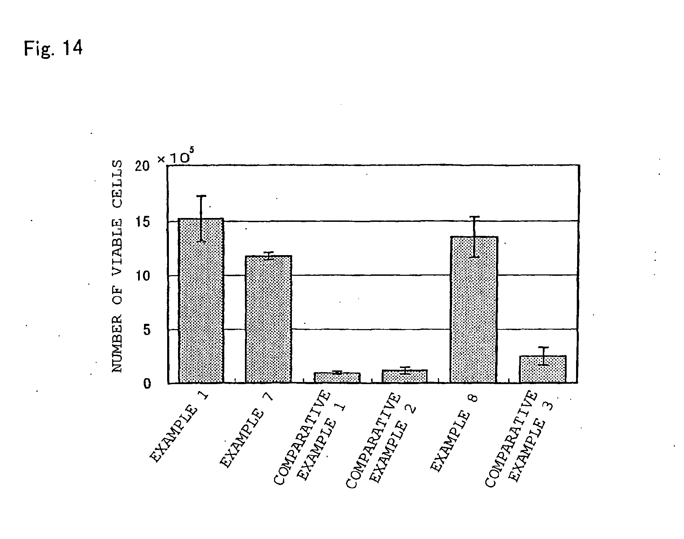

Fig. 14 is a graph of the number of viable cells measured in Example 1 and Examples 7 and 8 as well as Comparative Examples 1 to 3. In Example 1 and Examples 7 and 8 in which the cell layer was formed, the number of living cells was apparently great and it can be confirmed that the activity of cell growth was high.

### INDUSTRIAL APPLICABILITY OF THE INVENTION

According to the tissue equivalent for transplantation of the present invention, a large-size tissue equivalent for transplantation that can be transplanted in a short period and is excellent in the fusion ability with the surrounding tissues of the transplanted portion can be readily provided. As a result, it is particularly useful when the number of available cells is small or when a large tissue equivalent having a three-dimensional structure is needed promptly. Such a tissue equivalent 10 is excellent in the fusion ability with the surrounding tissues of the transplanted portion and the equivalent therefore may be fixed to the surrounding tissues of the transplanted portion comparatively promptly, so that the possibility that the tissue equivalent drops off can be decreased.

## Claims

1. A tissue equivalent for transplantation having a three-dimensional structure which is cultured *in vitro,* contains cells to be transplanted and which can be transplanted into a living body after the culture, **characterized by** comprising:
a scaffold layer mainly constituting a scaffold constructing the three-dimensional structure; and,
a cell layer which is localized at least in a part of the surface of the tissue equivalent for transplantation continuously with the scaffold layer and which contains a large amount of the cells to be transplanted or extra cellular matrix than the amount in the scaffold layer.

2. A tissue equivalent for transplantation according to claim 1, **characterized in that** the cell layer comprises the dense cells to be transplanted or further contains extra cellular matrix produced from the dense cells to be transplanted.

3. A tissue equivalent for transplantation according to claim 1, **characterized in that** the cell density of the cells to be transplanted existing in the cell layer is substantially dense compared with the cell density of the inside of the scaffold layer.

4. A tissue equivalent for transplantation according to claim 1, **characterized in that** the thickness of the cell layer is 20 to 500 µm.

5. A tissue equivalent for transplantation according to claim 1, **characterized in that** the scaffold is a bioabsorbable material or a bio-compatible material.

6. A tissue equivalent for transplantation according to claim 1, **characterized in that** the scaffold is one selected from the group consisting of collagen, hyaluronic acid, and polyrotaxane.

7. A tissue equivalent for transplantation according to claim 1, **characterized in that** the cell is one selected from the group consisting of chondrocyte, osteoblast, and progenitor cells thereof.

8. A method for producing a tissue equivalent for transplantation having a three-dimensional structure which is cultured *in vitro*, contains cells to be transplanted and which can be transplanted into a living body after the culture, **characterized by** comprising the steps of:
embedding cells to be transplanted in a scaffold constituting the three-dimensional structure;
supplying a medium in which the cells to be transplanted can be cultured on the scaffold in which the cells to be transplanted are embedded; and,
incubating the resultant under conditions where the proliferation ratio of the cells to be transplanted is higher on the surface of the scaffold than in the inside of the scaffold, so that the cell density of the cells to be transplanted becomes higher in at least a part of the surface of the scaffold than in the inside of the scaffold to thereby form two layers having a different cell density.

9. A method for producing a tissue equivalent for transplantation according to claim 8, **characterized in that** the medium contains ascorbic acid.

10. A method for producing a tissue equivalent for transplantation according to claim 9, **characterized in that** the medium containing ascorbic acid has been cryopreserved in a frozen state.

11. A method for producing a tissue equivalent for transplantation according to claim 8, **characterized in that** the cells to be transplanted are embedded in the scaffold with a seeding cell density of 1x10⁴ to 1x10⁸ cells/ml.

12. A method for producing a tissue equivalent for transplantation having a three-dimensional structure which is cultured *in vitro,* contains cells to be transplanted and which can be transplanted into a living body after the culture, **characterized by** comprising the steps of:
mixing a fluidity scaffold that can maintain a three-dimensional structure in a medium and cells to be transplanted;
seeding the mixture obtained by the mixing step on at least a part of the surface of a previously placed three-dimensional scaffold; and,
culturing until the cells to be transplanted become substantially dense in at least a part of the fluidity scaffold.
